(19) **Europäisches Patentamt**

**European Patent Office**

**Office européen des brevets**

(11)  **EP 1 054 029 B1**

(12)  **EUROPEAN PATENT SPECIFICATION**

(45) Date of publication and mention
of the grant of the patent:
**28.12.2005   Bulletin 2005/52**

(51) Int Cl.7: **C08G 18/02**

(21) Application number: **00303881.7**

(22) Date of filing: **09.05.2000**

(54) **Method of improving stability of aromatic polycarbodiimides**

Methode zur Verbesserung der Stabilität von aromatischen Polycarbodiimiden

Méthode pour améliorer la stabilité de polycarbodiimides aromatiques

(84) Designated Contracting States:
**DE FR GB IT NL SE**

(30) Priority:  **18.05.1999   US 134703 P**

(43) Date of publication of application:
**22.11.2000   Bulletin 2000/47**

(73) Proprietor: **ROHM AND HAAS COMPANY**
**Philadelphia, Pennsylvania 19106-2399 (US)**

(72) Inventor: **Brown, Ward Thomas**
**North Wales, Pennsylvania 19454 (US)**

(74) Representative: **Kent, Venetia Katherine et al**
**Rohm and Haas (UK) Ltd.,**
**European Patent Department,**
**28th Floor,**
**City Point,**
**One Ropemaker Street**
**London EC2Y 9HS (GB)**

(56) References cited:
**DE-A- 2 837 770        US-A- 3 755 242**
**US-A- 3 929 733        US-A- 4 085 140**
**US-A- 4 260 554**

## Description

This invention relates to a method of improving the stability of aromatic polycarbodiimides. More particularly, this invention relates to a method of improving the stability of aromatic polycarbodiimides using catalyst poisons.

Aromatic polycarbodiimides (hereinafter referred to as "ar-p CDI") are used as crosslinkers in a variety of applications, including coatings, adhesives, caulks, mastics. Generally, ar-pCDI are made by heating aromatic isocyanates in the presence of phosphorous oxides, usually phospholene oxides such as 3-methyl-1-phenyl-2-phospholene-1-oxide (hereinafter referred to as "MPPO"). The phosphorus oxides catalyze the reaction between 2 -NCO groups to make a carbodiimide (hereinafter referred to as "CDI") and $CO_2$:

$$2 \text{ R-NCO} \rightarrow \text{R-N=C=N-R} + CO_2$$

The catalyst reacts with -NCO to form a phosphinimide and carbon dioxide:

$$\text{R'NCO} + R_3\text{P=O} \rightarrow R_3\text{P=NR'} + CO_2$$

that then reacts with another R'NCO to regenerate the catalyst and make CDI:

$$R_3\text{P=NR'} + \text{R'NCO} \rightarrow \text{R'N=C=NR'} + R_3\text{P=O}.$$

After they are made, the ar-pCDI molecules begin to slowly build viscosity until they gel. While not wishing to be bound by theory, it is believed that the residual phosphorous oxide present in ar-pCDI solutions catalyze the reaction between the ar-pCDI and any compounds containing active hydrogens such as water, alcohols, amines, and other materials bearing active hydrogens that may contaminate the ar-pCDI solutions:

$$\text{N=C=N} + \text{ROH} \rightarrow \text{N=C(OR)NH}$$

Over time, the accumulation of N=C(OR)NH causes the ar-pCDI to build viscosity and eventually gel.

This instability limits the usefulness of ar-pCDI because the shelf life is too short, *i.e.,* less than six months, to be commercially viable. Aliphatic pCDI do not suffer the same stability problems as ar-pCDI, but, *inter alia,* are too reactive to make good crosslinkers for water-borne coatings when compared with ar-pCDI. Thus, ar-pCDI would be preferred if the shelf-life stability problems could be solved.

I have discovered that the stability or gel time of ar-p CDI is dependent on the level of phosphorous oxide used to make it and that remains in the ar-p CDI after manufacture; the more phosphorous oxide, the shorter the gel time. I have further discovered a way to deactivate these catalysts. Certain chemicals, hereinafter referred to as "catalyst poisons," may be added to the ar-pCDI after it is made that will react with the catalyst to deactivate it, thus greatly extending the storage stability or shelf-life of the ar-pCDI.

These catalyst poisons are not novel. For example in US-A-5,202,358, US-A-4,014,935, and US-A-4,614,785, organic isocyanates containing carbodiimides and/or uretone imine groups are prepared by the partial polymerization of the -NCO groups using phosphorous oxide catalysts. When only partial conversion takes place, the carbodiimides react with further isocyanate groups to give uretone imine groups. To ensure only a partial polymerization of the -NCO groups, US-A-5,202,358 discloses the addition of silylated acid compounds of the formula $X\text{-}[Si(CH_3)_3]_n$ to the reaction to terminate the formation of carbodiimide groups. To ensure only a partial polymerization of the -NCO groups, US-A-4,014,935 discloses that the phosphorous oxide catalyst is absorbed onto a substrate or deactivated by halides of hydrogen, tin, or phosphorus, or the oxyhalide of phosphorus or sulfur. To ensure only a partial polymerization of the -NCO groups, US-A-4,614,785 discloses that the phosphorus oxide catalyst is deactivated with sulfonyl isocyanates.

In each of the above-referenced patents, the starting material is an isocyanate catalyzed by a phosphorous oxide. Only a partial reaction is desired so that the final product is a mixture. However, the presence of the residual phosphorous oxide leads to an undesired, complete reaction. The deactivation of the catalyst, either by adsorption onto a substrate or by the addition of a catalyst poison, prevents complete conversion to the pCDI. The present invention utilizes the deactivation steps but their purpose is to prevent the residual phosphorous oxide in the completed reaction from gelling the system rather from preventing the completed reaction.

## Statement of the Invention

The present invention is as set out in the accompanying claims.

The invention is directed to a method of improving the stability of ar-pCDI formed using a phosphorous oxide catalyst including the step of deactivating the catalyst.

Preparation of ar-pCDI using phosphorous oxide catalysts may be by conventional means such as those methods disclosed in US-A-2,853,473, US-A-2,941,966, US-A-2,941,983, US-A-4,487,964 and US-A-5,574,083. On completion of the preparation of ar-pCDI, the phosphorous oxide catalysts is deactivated by addition to the ar-pCDI of at least one catalyst poison.

Deactivation may be carried out by addition to the ar-pCDI of at least one catalyst poison. The catalyst poisons useful in the method of invention are silylated acids of the formula $X-[Si(CH_3)_3]_n$ where X represents the neutral acid residue obtained by removal of the acidic hydrogen atoms from an n-basic acid having a pKa value of at most 3, other than a hydrohalic acid, and n is an integer of 1 to 3.

Examples of suitable silylated acids of the formula $X-[Si(CH_3)_3]_n$ include silylated sulfonic acids, such as trifluoromethanesulfonic acid trimethyl silyl ester and methanesulfonic acid trimethylsilyl ester; and silylated esters of acids of phosphorous, such as phosphoric acid tris(trimethylsilyl ester) and phosphoric acid diethyl ester trimethylsilyl ester.

The catalyst poison may be added to the ar-pCDI at a molar ratio of 0.1:1 to 30:1 (catalyst poison:phosphorus oxide catalyst), preferably, 0.3:1 to 5:1. The catalyst poison may be added neat or as a solution in a suitable solvent.

Compositions containing ar-pCDI made by the method of the present invention are more stable to storage at room temperature, especially up to 6 months, and are more thermally stable at elevated temperatures, for example of up to 80-120°C than those compositions containing ar-pCDI made by conventional methods.

Some embodiments of the present invention will now be described in detail in the following Examples.

## Examples

## Example 1

A 250 ml round bottom flask was outfitted with a magnetic stirrer, $N_2$ sparge tube, reflux condenser, and thermocouple. The flask was charged with 47.88 g tolylene diisocyanate (mixture of isomers; 80% 2,4-tolylene diisocyanate and 20% 2,6-tolylene diisocyanate), 32.2 g polyethylene glycol monomethyl ether (average molecular weight. = 350 g/mole), and 5.0 g propylene glycol methyl ether acetate. Flask was flushed with $N_2$, then sparge rate set to 5 ml/min and flask heated to 80°C for 1 hour. 5.45 g of a 2.04% solution of MPPO in propylene glycol methyl ether acetate and 59.6 g of propylene glycol methyl ether acetate was added and the flask heated to 120°C for 3.25 hours. One hour into heating the flask at 120°C the $N_2$ sparge was increased to 20 ml/min. The ar-pCDI solution was allowed to cool to room temperature. An IR spectrum taken of the product showed no residual -NCO groups. The ar-pCDI solution was placed in a series of glass vials and catalyst poisons were added as indicated in Table 1. Vials A and B were then placed on a hot plate and heated for 15 minutes on the hot plate's "high" setting. The vials were observed for an increase in viscosity, and the time to gelation was recorded. These results are also given in Table 1.

Table 1

| Vial ID | Level of ar-pCDI solution (g) | Catalyst poison | Level of catalyst poison (g) | Moles poison: moles MPPO | Heated | Days to gel |
|---|---|---|---|---|---|---|
| A* | 20.54 | chlorosulfonyl isocyanate | 0.0229 | 2.1:1 | Yes | > 455 |
| B** | 19.38 | phosphoric acid tris(trimethyl-silyl ester) | 0.0306 | 1.3:1 | Yes | > 455 |
| C* | 20.40 | chlorosulfonyl isocyanate | 0.0184 | 1.7:1 | No | > 455 |

*Comparative example

** Example of the invention

Table 1   (continued)

| Vial ID | Level of ar-pCDI solution (g) | Catalyst poison | Level of catalyst poison (g) | Moles poison: moles MPPO | Heated | Days to gel |
|---|---|---|---|---|---|---|
| D* | 22.12 | chlorosulfonyl isocyanate (33.7% solution in xylene) | 0.0137 | 1.1:1 | No | > 455 |
| E** | 21.39 | phosphoric acid tris(trimethyl-silyl ester) | 0.0326 | 1.3:1 | No | > 455 |
| F* | 21.99 | - | - | 0 | No | 173 |
| G* | 10.01 | chlorosulfonyl isocyanate (33.7% solution in xylene) | 0.0019 | 0.35:1 | No | > 455 |

*Comparative example

** Example of the invention

Those ar-pCDI to which a catalyst poison had been added (Vials A, B, C, D, E and G) showed significant improvement in time to gel compared to the ar-pCDI to which catalyst poison had not been added (Vial F).

**Claims**

1. A method of improving the stability of aromatic polycarbodiimide formed using at least one phospholene oxide catalyst: **characterized in that**, on completion of the preparation of the aromatic polycarbodiimide reaction, the residual phospholene oxide catalyst is deactivated by addition to the aromatic polycarbodiimide of at least one catalyst poison selected from the group consisting of silylated acids of the formula $X\text{-}[Si(CH_3)_3]_n$, where X represents the neutral acid residue obtained by removal of the acidic hydrogen atoms from an n-basic acid having a pKa value of at most 3, other than a hydrohalic acid, and a is an integer of 1 to 3.

2. The method of claim 1 wherein said phospholene oxide catalyst is 3-methyl-1-phenyl-2-phospholene-1-oxide.

3. The method of claim 1 wherein said catalyst poison is added to said ar-pCDI at a molar ratio of 0.1:1 to 30:1 (catalyst poison: phospholene oxide catalyst).

4. The method of claim 1 wherein said catalyst poison is a silylated ester of acids of phosphorous.

5. The method of claim 1 wherein said catalyst poison is phosphoric acid tris(trimethyl-silyl ester).

**Patentansprüche**

1. Verfahren zur Verbesserung der Stabilität eines aromatischen Polycarbodiimids, das unter Verwendung mindestens eines Phospholenoxid-Katalysators hergestellt worden ist, **dadurch gekennzeichnet, dass** nach Vervollständigung der Durchführung der aromatischen Polycarbodiimid-Reaktion der verbleibende Phospholenoxid-Katalysator durch Zugabe mindestens eines Katalysatorgiftes, ausgewählt aus der Gruppe, bestehend aus silylierten Säuren der Formel $X\text{-}[Si(CH_3)_3]_n$, wobei X einen neutralen Säurerest darstellt, der durch Entfernen der aciden Wasserstoffatome von einer n-basigen Säure mit einem pKa-Wert von höchstens 3, welche von einer Halogenwasserstoffsäure verschieden ist, erhalten wird, und wobei n eine ganze Zahl von 1 bis 3 ist, zu dem aromatischen Polycarbodiimid deaktiviert wird.

2. Verfahren nach Anspruch 1, wobei der Phospholenoxid-Katalysator 3-Methyl-1-phenyl-2-phospholen-1-oxid ist.

3. Verfahren nach Anspruch 1, wobei das Katalysatorgift zu dem ar-pCDI in einem molaren Verhältnis von 0,1:1 bis

30:1 (Katalysatorgift : Pholsphenoxid-Katalysator) gegeben wird.

**4.** Verfahren nach Anspruch 1, wobei das Katalysatorgift ein silylierter Ester von Säuren des Phosphors ist.

**5.** Verfahren nach Anspruch 1, wobei das Katalysatorgift Phosphorsäure-tris(trimethylsilylester) ist.

**Revendications**

**1.** Procédé pour améliorer la stabilité d'un polycarbodiimide aromatique formé au moyen d'au moins un catalyseur oxyde de phospholène : **caractérisé en ce que**, à l'achèvement de la réaction de préparation du polycarbodiimide aromatique, le catalyseur oxyde de phospholène résiduel est désactivé par addition au polycarbodiimide aromatique d'au moins un poison de catalyseur choisi dans le groupe consistant en les acides silylés de formule X-[Si$(CH_3)_3]_n$ où X représente le résidu d'acide neutre obtenu par retrait des atomes d'hydrogène acides d'un acide n fois fonctionnel ayant une valeur de pKa d'au plus 3, différent d'un acide halogénhydrique, et n est un entier de 1 à 3.

**2.** Procédé selon la revendication 1 où ledit catalyseur oxyde de phospholène est le 3-méthyl-1-phényl-2-phospholène-1-oxyde.

**3.** Procédé selon la revendication 1 où ledit poison de catalyseur est ajouté audit ar-pCDI à un rapport molaire de 0,1:1 à 30:1 (poison de catalyseur : catalyseur oxyde de phospholène).

**4.** Procédé selon la revendication 1 où ledit poison de catalyseur est un ester silylé d'acides du phosphore.

**5.** Procédé selon la revendication 1 où ledit poison de catalyseur est le tris(triméthyl-silylester) d'acide phosphorique.